(19) Europäisches Patentamt
European Patent Office
Office européen des brevets

(11) EP 4 738 211 A1

(12) # EUROPEAN PATENT APPLICATION

(43) Date of publication:
**06.05.2026 Bulletin 2026/19**

(21) Application number: **25210938.4**

(22) Date of filing: **24.10.2025**

(51) International Patent Classification (IPC):
***G06Q 10/04*** *(2023.01)* ***G16C 20/10*** *(2019.01)*

(52) Cooperative Patent Classification (CPC):
**G16C 20/10; G06Q 10/04**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **28.10.2024 JP 2024188747**

(71) Applicant: **FUJITSU LIMITED**
**Kawasaki-shi, Kanagawa 211-8588 (JP)**

(72) Inventor: **HIGUCHI, Hiroyuki**
**Kawasaki-shi, Kanagawa, 211-8588 (JP)**

(74) Representative: **Hoffmann Eitle**
**Patent- und Rechtsanwälte PartmbB**
**Arabellastraße 30**
**81925 München (DE)**

(54) **ANALYSIS PROGRAM, ANALYSIS DEVICE, AND ANALYSIS METHOD**

(57) An analysis program that causes a computer to execute a process includes first extracting (S201), from a set of data including a value of each of a plurality of explanatory variables and a value of an objective variable, a specific condition having a specific correlation with the objective variable among conditions for at least a part of the plurality of explanatory variables, second extracting (S202) a set of combinations of a value of a predetermined explanatory variable and a value of the objective variable indicated by predetermined variable relationship information from the set of data based on the specific condition, dividing (S203) the set of combinations into a first group and a second group, and comparing (S204) positive and negative signs of a first coefficient indicating a relationship between the predetermined explanatory variable and the objective variable represented by a combination of the first group with positive and negative signs of a second coefficient indicating a relationship between the predetermined explanatory variable and the objective variable represented by a combination of the second group, wherein the predetermined variable relationship information indicates a relationship between the predetermined explanatory variable and the objective variable in data processing similar to data processing on the set of data.

FIG.1

101

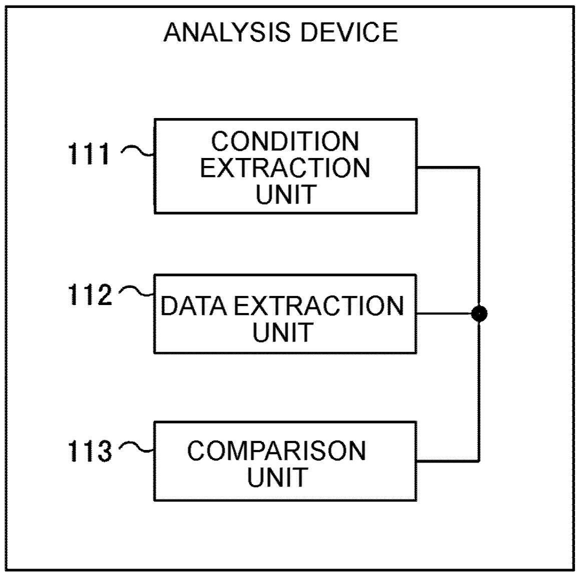


EP 4 738 211 A1

## Description

### FIELD

The embodiments discussed herein are related to an analysis program, an analysis device, and an analysis method.

### BACKGROUND

In a material search for an optimal material to be used for production of a product, a scatter diagram called a volcano plot may be used. The horizontal and vertical axes of the volcano plot represent the properties with respect to the material.

As an example of material search using a volcano plot, research on a catalyst for electrochemical ammonia synthesis is known (See, for example, E. Drazevic et al., "Are There Any Overlooked Catalysts for Electrochemical NH3 Synthesis-New Insights from Analysis of Thermochemical Data", iScience, Volume 23, Issue 12, 33 pages, December 18, 2020.).

The horizontal axis of a volcano plot corresponds to an explanatory variable, and the vertical axis corresponds to an objective variable. The shape of the graph of the volcano plot is a mountain shape or a valley shape, and the relationship between the explanatory variable and the objective variable significantly changes at the vertex of the graph. In the case of material search, a volcano plot is often used because a feature of a desired material can be objectively understood from values of an explanatory variable and an objective variable at a vertex of a graph.

However, when a volcano plot is generated from data including a combination of many properties related to a material, selection of a property to be used as an explanatory variable largely depends on knowledge and experience of experts. In addition, it is not necessarily easy to determine the possibility of generating a volcano plot from a scatter diagram in which explanatory variables and objective variables are plotted.

Note that such a problem occurs not only when a set of data is analyzed for material search but also when a set of data is analyzed for various purposes.

Accordingly, it is an object in one aspect of an embodiment of the invention to provide an analysis program, an analysis device, and an analysis method that efficiently determines a relationship between two variables from a set of data including values of a plurality of variables.

### SUMMARY

According to an aspect of an embodiment, an analysis program that causes a computer to execute a process including extracting, from a set of data including a value of each of a plurality of explanatory variables and a value of an objective variable, a specific condition having a specific correlation with the objective variable among conditions for at least a part of the plurality of explanatory variables; extracting a set of combinations of a value of a predetermined explanatory variable and a value of the objective variable indicated by predetermined variable relationship information from the set of data based on the specific condition; dividing the set of combinations into a first group and a second group; and comparing positive and negative signs of a first coefficient indicating a relationship between the predetermined explanatory variable and the objective variable represented by a combination of the first group with positive and negative signs of a second coefficient indicating a relationship between the predetermined explanatory variable and the objective variable represented by a combination of the second group; and the predetermined variable relationship information indicates a relationship between the predetermined explanatory variable and the objective variable in data processing similar to data processing on the set of data.

### BRIEF DESCRIPTION OF DRAWINGS

FIG. 1 is a functional configuration diagram of an analysis device according to an embodiment;
FIG. 2 is a flowchart of first analysis processing;
FIG. 3 is a functional configuration diagram illustrating a specific example of the analysis device;
FIGS. 4A and 4B are diagrams illustrating first variable relationship information;
FIGS. 5A and 5B are diagrams illustrating second variable relationship information;
FIG. 6 is a diagram illustrating second analysis processing;
FIG. 7 is a diagram illustrating an initial atomic structure;
FIG. 8 is a diagram illustrating a data set;
FIG. 9 is a diagram illustrating a volcano plot;
FIG. 10 is a diagram illustrating a causal graph;
FIG. 11A is a flowchart (part 1) of the second analysis processing;
FIG. 11B is a flowchart (part 2) of the second analysis processing; and
FIG. 12 is a hardware configuration diagram of an information processing device.

### DESCRIPTION OF EMBODIMENTS

Preferred embodiments of the present invention will be explained with reference to accompanying drawings. Note that the analysis program, the analysis device, and the analysis method disclosed in the present application are not limited to the following examples.

FIG. 1 illustrates a functional configuration example of an analysis device according to an embodiment. An analysis device 101 of FIG. 1 includes a condition extraction unit 111, a data extraction unit 112, and a comparison unit 113.

FIG. 2 is a flowchart illustrating an example of first analysis processing performed by the analysis device 101 of FIG. 1. First, the condition extraction unit 111 extracts a specific condition having a specific correlation with an objective variable among conditions that are a combination of a plurality of explanatory variables from a set of data including a value of each of the plurality of explanatory variables and a value of the objective variable (step 201).

Next, the data extraction unit 112 extracts a set of combinations of the value of a predetermined explanatory variable and the value of the objective variable indicated by predetermined variable relationship information from the set of data based on the specific condition (step 202). The predetermined variable relationship information indicates a relationship between the predetermined explanatory variable and the objective variable in data processing similar to data processing for the set of data.

Next, the comparison unit 113 divides the set of combinations into a first group and a second group (step 203), and compares the positive and negative signs of a first coefficient with the positive and negative signs of a second coefficient (step 204). The first coefficient indicates a relationship between a predetermined explanatory variable and the objective variable represented by a combination of the first group, and the second coefficient indicates a relationship between a predetermined explanatory variable and the objective variable represented by a combination of the second group.

With the analysis device 101 of FIG. 1, a relationship between two variables can be efficiently determined from a set of data including values of a plurality of variables.

FIG. 3 illustrates a specific example of the analysis device 101 of FIG. 1. An analysis device 301 in FIG. 3 includes a first generation unit 311, a causal estimation unit 312, an extraction unit 313, a second generation unit 314, an output unit 315, and a storage unit 316.

The causal estimation unit 312, the extraction unit 313, and the second generation unit 314 correspond to the condition extraction unit 111, the data extraction unit 112, and the comparison unit 113 in FIG. 1, respectively.

The analysis device 301 analyzes data in the material search. The analysis device 301 generates data on physical properties of a material, for example, by simulating an electrochemical reaction of the material, and generates a volcano plot indicating a relationship between properties of the material by analyzing the generated data.

The storage unit 316 stores accumulated data 321 including a plurality of pieces of variable relationship information. Each piece of variable relationship information is, for example, a volcano plot or a conditional causal graph indicating a relationship between an explanatory variable and an objective variable in an electrochemical reaction of a material. The explanatory variable and the objective variable represent the property of the material.

In the accumulated data 321, each piece of variable relationship information is associated with the type of data processing. The type of data processing includes the type of reaction in the material search and the type of search target. For example, when searching for a catalyst material for electrochemical ammonia synthesis, the type of reaction is electrochemical ammonia synthesis, and the type of search target is a catalyst.

The variable relationship information included in the accumulated data 321 is acquired, for example, by performing simulation of a chemical reaction, an experiment, or the like. The variable relationship information may be a volcano plot described in a literature such as a paper on a chemical reaction.

FIGS. 4A and 4B illustrate an example of the first variable relationship information included in the accumulated data 321. FIG. 4A illustrates an example of a first volcano plot in electrochemical ammonia synthesis. The horizontal axis represents the adsorption energy $\Delta E_N$ (eV) of the nitrogen atom N, and the vertical axis represents the limiting potential LP (V). $\Delta E_N$ corresponds to an explanatory variable, and LP corresponds to an objective variable.

The shape of the volcano plot in FIG. 4A is a mountain shape, and the coordinates of the vertex are (-0.9, -0.3). The slope k1 of the straight line in the section of $\Delta E_N < -0.9$ is 0.5, and the slope k2 of the straight line in the section of $\Delta E_N \geq -0.9$ is -0.78. Therefore, this volcano plot can be recorded using six pieces of information: variable name $\Delta E_N$, variable name LP, 0.5, -0.78, -0.9, and -0.3. The value -0.9 of $\Delta E_N$ at the vertex represents a threshold for $\Delta E_N$.

FIG. 4B illustrates an example of a first conditional causal graph. The volcano plot of FIG. 4A can be represented using the conditional causal graph of FIG. 4B. The causal graph includes a plurality of nodes representing a cause or a result in the causal relationship and an edge from the node representing the cause to the node representing the result. A causal effect, which is an index indicating the strength of the influence of the cause on the result, is given to each edge. The causal effect is an example of an index indicating the strength of the causal relationship.

$\Delta E_N$ represents the cause and LP represents the result. The relationship in the section of $\Delta E_N < -0.9$ is represented by using a node representing $\Delta E_N$, a node representing LP, and an edge from $\Delta E_N$ to LP. The edge from $\Delta E_N$ to LP is given 0.5, which is a slope of a straight line in the section of $\Delta E_N < -0.9$, as a causal effect.

The relationship in the section of $\Delta E_N \geq -0.9$ is also represented by using a node representing $\Delta E_N$, a node representing LP, and an edge from $\Delta E_N$ to LP. The edge from $\Delta E_N$ to LP is given -0.78, which is a slope of a straight line in the section of $\Delta E_N \geq -0.9$, as a causal effect.

FIGS. 5A and 5B illustrate an example of the second variable relationship information included in the accumulated data 321. FIG. 5A illustrates an example of a second volcano plot in electrochemical ammonia synthesis. The horizontal axis represents the adsorption energy $\Delta E_{NNH}$ (eV) of the intermediate product NNH including two nitrogen atoms N and one hydrogen atom H, and the vertical axis represents the limiting potential LP (V). $\Delta E_{NNH}$ corresponds to an explanatory variable, and LP corresponds to an objective variable.

The shape of the volcano plot in FIG. 5A is a mountain shape, and the coordinates of the vertex are (-0.72, -0.2). The slope k1 of the straight line in the section of $\Delta E_{NNH}$ < -0.72 is 0.65, and the slope k2 of the straight line in the section of $\Delta E_{NNH} \geq$ -0.72 is -0.98. Therefore, this volcano plot can be recorded using six pieces of information: variable name $\Delta E_{NNH}$, variable name LP, 0.65, -0.98, -0.72, and -0.2. The value -0.72 of $\Delta E_{NNH}$ at the vertex represents a threshold for $\Delta E_{NNH}$.

FIG. 5B illustrates an example of a second conditional causal graph. The volcano plot of FIG. 5A can be represented using the conditional causal graph of FIG. 5B. $\Delta E_{NNH}$ represents the cause and LP represents the result. The relationship in the section of $\Delta E_{NNH}$ < -0.72 is represented by using a node representing $\Delta E_{NNH}$, a node representing LP, and an edge from $\Delta E_{NNH}$ to LP. The edge from $\Delta E_{NNH}$ to LP is given 0.65, which is a slope of a straight line in the section of $\Delta E_{NNH}$ < -0.72, as a causal effect.

The relationship in the section of $\Delta E_{NNH} \geq$ -0.72 is also represented by using a node representing $\Delta E_{NNH}$, a node representing LP, and an edge from $\Delta E_{NNH}$ to LP. The edge from $\Delta E_{NNH}$ to LP is given -0.98, which is a slope of a straight line in the section of $\Delta E_{NNH} \geq$ -0.72, as a causal effect.

FIG. 6 illustrates an example of second analysis processing performed by the analysis device 301 of FIG. 3. In the analysis processing of FIG. 6, data in material search for searching for a catalyst material for electrochemical ammonia synthesis is analyzed.

The first generation unit 311 generates a data set 322 indicating a simulation result by performing simulation of electrochemical ammonia synthesis at an atomic level using the atomic structure of the material, and stores the data set in the storage unit 316.

FIG. 7 illustrates an example of an initial atomic structure in a simulation of electrochemical ammonia synthesis. The atomic structure in FIG. 7 contains a metal atom 701 as a catalyst and a nitrogen atom 702 as an adsorbate, and is expressed by using the kind of element of each atom and three-dimensional coordinates.

FIG. 8 illustrates an example of the data set 322 indicating a simulation result of electrochemical ammonia synthesis. Each row corresponds to one piece of data, and includes an element, an atomic number, a period number, a group number, $\Delta E_N$, and LP.

The element represents the type of the metal atom 701, the atomic number represents the atomic number of the metal atom 701, the period number represents the period of the metal atom 701 in the periodic table, and the group number represents the group of the metal atom 701 in the periodic table. $\Delta E_N$ represents the adsorption energy of the nitrogen atom 702, and LP represents the limiting potential.

The element, atomic number, period number, group number, and $\Delta E_N$ correspond to explanatory variables, and LP corresponds to an objective variable. The data of each row is an example of data including the value of each of the plurality of explanatory variables and the value of the objective variable.

The causal estimation unit 312 generates a condition representing each of a plurality of combinations of explanatory variables by comprehensively combining the explanatory variables included in the data set 322. As an example, a case where the explanatory variables X1 to Xn (n is an integer of 1 or more) and the objective variable Y are included in the data set 322 will be described. The causal estimation unit 312 multi-levels each explanatory variable Xi (i = 1 to n).

For example, in a case where the value of the explanatory variable X1 is a numerical value, the causal estimation unit 312 can multi-level the explanatory variable X1 by dividing the range of the numerical value into m+1 sections using the threshold T1 to the threshold Tm (m is an integer of 1 or more). The m+1 sections are represented by $X1 \leq T1$, $T1 < X1 \leq T2$, $T2 < X1 \leq T3$, ..., $T(m-1) < X1 \leq Tm$, and $Tm < X1$.

Furthermore, in a case where the category value of the explanatory variable X9 is 0 or 1, the causal estimation unit 312 can multi-level the explanatory variable X9 as X9 = 0 and X9 = 1. The number of category values may be three or more.

Then, the causal estimation unit 312 generates a plurality of conditions including, for example, the following conditions 1 to 4 as conditions for one or a plurality of explanatory variables Xi.

$$\text{Condition 1: } X1 > T1$$

$$\text{Condition 2: } X1 > T1 \land X2 > T2$$

$$\text{Condition 3: } X3 > T3$$

$$\text{Condition 4: } X3 > T3 \land X4 > T2$$

"$\land$" represents a logical product. Here, the conditions other than condition 1 to condition 4 are omitted.

Next, the causal estimation unit 312 extracts a condition having a correlation with the objective variable Y from the generated conditions. For example, the causal estimation unit 312 obtains the absolute value of the correlation coefficient between each explanatory variable Xi and the objective variable Y with respect to the subset of data satisfying the condition, and in a case where there is the explanatory variable Xi in which the absolute value of the correlation coefficient is equal to or greater than the threshold, it is determined that the condition has the correlation with the objective variable Y. The condition having the correlation with the objective variable Y is an example of a condition for at least a part of the plurality of explanatory variables.

For example, when there is an explanatory variable in which the absolute value of the correlation coefficient with the objective variable Y is equal to or greater than the threshold in the condition 1 and the condition 2, a plurality of conditions including the condition 1 and the condition 2 among a plurality of conditions including the conditions 1 to 4 are extracted as conditions having a correlation with the objective variable Y.

Next, the causal estimation unit 312 generates a causal graph for each condition having a correlation with the objective variable Y. For example, the causal estimation unit 312 extracts a subset of data in which the value of the explanatory variable Xi satisfies a condition from the data set 322, performs statistical causal estimation using the extracted subset, and generates a causal graph for the condition. A causal effect estimated by statistical causal estimation is given to each edge of the causal graph.

Next, the causal estimation unit 312 compares the causal effect given to the edge toward the objective variable Y of each causal graph with a predetermined reference value. In a case where the causal effect of any one of the causal graphs is equal to or greater than the reference value, the causal estimation unit 312 extracts a condition corresponding to the causal graph as the specific condition 323 and stores the extracted condition in the storage unit 316.

In a case where the causal effect given to the edge toward the objective variable Y is equal to or greater than the reference value, by extracting the condition corresponding to the causal graph, it is possible to select a condition that brings about a strong causal effect to the objective variable Y from among conditions having a correlation with the objective variable Y.

The causal effect given to the edge toward the objective variable Y is an example of an index indicating the strength of the causal relationship between any of the plurality of explanatory variables and the objective variable. The specific condition 323 is an example of a specific condition having a specific correlation with the objective variable.

The extraction unit 313 selects variable relationship information associated with data processing similar to the data processing for the data set 322 from the variable relationship information included in the accumulated data 321 as the similar variable relationship information 324, and stores the same in the storage unit 316. For example, the extraction unit 313 selects variable relationship information associated with the same type as the type of data processing for the data set 322 as the similar variable relationship information 324.

When two pieces of data processing are similar to each other, variable relationship information generated in the two pieces of data processing is often similar to each other. Therefore, by referring to the similar variable relationship information 324 in the similar data processing, the variable relationship information for the data set 322 can be generated using the information such as the variable included in the similar variable relationship information 324.

The similar variable relationship information 324 is an example of predetermined variable relationship information, and the explanatory variable included in the similar variable relationship information 324 is an example of a predetermined explanatory variable. Hereinafter, the explanatory variable included in the similar variable relationship information 324 may be referred to as an explanatory variable P.

Next, the extraction unit 313 extracts, from the data set 322, a subset of data that satisfies a condition for an explanatory variable other than the explanatory variable P included in the similar variable relationship information 324 among the explanatory variables included in the specific condition 323.

For example, when the condition 2 is extracted as the specific condition 323 and the explanatory variable P included in the similar variable relationship information 324 is X1, the explanatory variable other than the explanatory variable P among the explanatory variable X1 and the explanatory variable X2 included in the specific condition 323 is X2. Therefore, a subset of data including a value of X2 satisfying $X2 > T2$ that is a part of Condition 2 is extracted from the data set 322.

Next, the extraction unit 313 extracts a combination of the value of the explanatory variable P and the value of the objective variable from each data included in the extracted subset. Then, the extraction unit 313 stores a set of combinations extracted from each of the plurality of pieces of data in the storage unit 316 as a variable value set 325. The variable value set 325 is an example of the set of combinations of a value of a predetermined explanatory variable and a value of an objective variable.

By extracting a combination of the value of the explanatory variable P and the value of the objective variable from each data satisfying the condition for the explanatory variable other than the explanatory variable P, the variable value set 325 including a combination of variable values suitable for generation of the volcano plot can be generated.

The second generation unit 314 divides the variable value set 325 into a group G1 and a group G2 by using a threshold Ta of the explanatory variable P included in the specific condition 323. The threshold Ta is one of thresholds used to multi-level the explanatory variable P when the value of the explanatory variable P is a numerical value.

For example, the second generation unit 314 classifies a combination including a value less than Ta as the value of the explanatory variable P into the group G1, and classifies a combination including a value equal to or greater than Ta as the value of the explanatory variable P into the group G2.

The group G1 is an example of a first group, and the group G2 is an example of a second group.

Next, the second generation unit 314 plots points representing each combination included in the group G1 and the group G2 on the XY plane in which the explanatory variable P is the X axis and the objective variable is the Y axis. Then, the second generation unit 314 obtains a regression line from the plotted points for each of the group G1 and the group G2, and calculates the slope of the regression line as a regression coefficient.

The regression coefficient of the group G1 is an example of a first coefficient indicating the relationship between the predetermined explanatory variable and the objective variable represented by the combination of the first group. The regression coefficient of the group G2 is an example of a second coefficient indicating the relationship between the predetermined explanatory variable and the objective variable represented by the combination of the second group.

Next, the second generation unit 314 compares the positive and negative signs of the regression coefficient of the group G1 with the positive and negative signs of the regression coefficient of the group G2.

When the positive and negative signs of the regression coefficient of the group G1 are different from the positive and negative signs of the regression coefficient of the group G2, the second generation unit 314 determines that a volcano plot can be generated. Therefore, the second generation unit 314 uses the variable value set 325 to generate the volcano plot 326 representing the relationship between the explanatory variable P and the objective variable in the data processing on the data set 322, and stores the same in the storage unit 316. The volcano plot 326 is an example of specific variable relationship information.

On the other hand, when the positive and negative signs of the regression coefficient of the group G1 are the same as the positive and negative signs of the regression coefficient of the group G2, the second generation unit 314 determines that the volcano plot 326 cannot be generated.

By generating the volcano plot 326 immediately when the positive and negative signs of the regression coefficients of the group G1 are different from the positive and negative signs of the regression coefficients of the group G2, the volcano plot 326 including the explanatory variable P can be generated in a short time. In addition, by using the volcano plot or the conditional causal graph as the variable relationship information included in the accumulated data 321, the volcano plot 326 can be efficiently generated using the similar variable relationship information 324.

FIG. 9 illustrates an example of the volcano plot 326 generated using the variable value set 325. The X-axis represents the adsorption energy $\Delta E_N$ (eV) of the nitrogen atom N, and the Y-axis represents the limiting potential LP (V). $\Delta E_N$ corresponds to the explanatory variable P, and LP corresponds to an objective variable. Each plotted point represents a combination of the value of the explanatory variable P and the value of the objective variable. In this example, the data set 322 of FIG. 8 is used, and the volcano plot of FIG. 4A is used as the similar variable relationship information 324.

The slope k1 of a regression line 901 of the group G1 is 0.23, and the slope k2 of a regression line 902 of the group G2 is -1.7. Therefore, the positive and negative signs of the slope k2 are reversed from the positive and negative signs of the slope k1. Therefore, the second generation unit 314 detects an intersection of the regression line 901 and the regression line 902 as a vertex, and generates the volcano plot 326.

The shape of the volcano plot 326 in FIG. 9 is a mountain shape, and the coordinates of the vertex are (-0.75, -0.53). Therefore, this volcano plot 326 includes six pieces of information of variable name $\Delta E_N$, variable name LP, 0.23, -1.7, -0.75, and -0.53. Similarly, when the shape of the volcano plot 326 is the valley type, the vertex is detected from the regression line, and the volcano plot 326 is generated.

Next, the second generation unit 314 compares the volcano plot 326 with the similar variable relationship information 324 to generate the explanatory information 327 related to the difference between the volcano plot 326 and the similar variable relationship information 324, and stores the explanatory information in the storage unit 316. The second generation unit 314 can generate the explanatory information 327 using, for example, the causal graph generated by the causal estimation unit 312.

For example, comparing the volcano plot 326 of FIG. 9 with the volcano plot of FIG. 4A, the coordinates of the vertex change from (-0.9, -0.3) to (-0.75, -0.53). Therefore, the second generation unit 314 generates the explanatory information 327 with reference to the causal graph including $\Delta E_N$ and LP.

FIG. 10 illustrates an example of a causal graph including $\Delta E_N$ and LP. The causal graph in FIG. 10 includes a node representing an interatomic distance, a node representing $\Delta E_N$, a node representing LP, an edge from the interatomic distance toward $\Delta E_N$, and an edge from the interatomic distance toward LP. The interatomic distance represents the cause and $\Delta E_N$ and LP represent the result.

A causal effect -1 is imparted to the edge from the interatomic distance toward $\Delta E_N$, and a causal effect 2 is imparted to the edge from the interatomic distance toward LP. Therefore, as the interatomic distance increases, $\Delta E_N$ decreases and LP increases. Further, as the interatomic distance decreases, $\Delta E_N$ increases and LP decreases.

The element included in each data of the data set 322 illustrated in FIG. 8 represents a single metal used as a catalyst. For example, when it is known that the volcano plot of FIG. 4A is generated from a data set using a metal nitride as a catalyst, it can be seen that the interatomic distance is reduced by changing the catalyst to be searched from the metal nitride to a single metal. Then, from the causal graph in FIG. 10, it can be explained that as a result of the decrease in the interatomic distance, $\Delta E_N$ at the vertex increases and LP at the vertex decreases.

In this case, the explanatory information 327 indicating that the difference between the volcano plot 326 and the similar variable relationship information 324 is caused by the fact that the catalyst of the volcano plot 326 is a single metal and the catalyst of the similar variable relationship information 324 is a metal nitride is generated.

The output unit 315 outputs the generated volcano plot 326 and the explanatory information 327.

With the analysis device 301 of FIG. 3, the explanatory variable P used for generating the volcano plot 326 is specified using the similar variable relationship information 324 corresponding to data processing similar to the data processing on the data set 322. Then, it is determined whether or not there is a possibility to generate the volcano plot 326 including the explanatory variable P using the threshold Ta of the explanatory variable P included in the specific condition 323.

When it is determined whether or not there is a possibility to generate a volcano plot without using the similar variable relationship information 324, the determination is repeated exhaustively for all the explanatory variables included in the specific condition 323, so that the processing time required for the determination increases.

On the other hand, there is a high possibility that the volcano plot 326 for the data set 322 is generated by using the explanatory variable P included in the similar variable relationship information 324 of the similar data processing.

Therefore, it is not necessary to perform determination on all the explanatory variables included in the specific condition 323, and the relationship between the explanatory variable P and the objective variable included in the data set 322 can be efficiently determined.

As a result, the processing time required for determining whether or not the volcano plot 326 can be generated is shortened, so that the processing of generating the volcano plot 326 for the data set 322 is sped up. Therefore, the efficiency of the analysis processing in the material search is improved, and shortening of the development period and cost reduction are achieved.

In addition, by generating the accumulated data 321 including a plurality of pieces of variable relationship information in advance, it is possible to reduce time and effort for searching and confirming a volcano plot generated by similar data processing from a past paper or the like.

Furthermore, by outputting the explanatory information 327 and presenting the explanatory information to the user, it becomes easy to understand the difference between the volcano plot 326 and the similar variable relationship information 324 and the cause thereof. As a result, it is possible to reduce time and effort to compare the volcano plot 326 with the similar variable relationship information 324, extract a difference, and examine the cause thereof.

The analysis device 301 of FIG. 3 can also analyze data in other data processing other than material search to generate the volcano plot 326. The other data processing is, for example, data processing for generating a promotion measure for a customer in a marketing operation. The other data processing may be data processing for generating measures for solving problems in other operations such as manufacturing and medical care.

The explanatory variable P included in the similar variable relationship information 324 is not necessarily included in the data set 322. When the value of the explanatory variable P is not included in the data set 322, the analysis device 301 performs processing of updating the data set 322 so that data including the value of the explanatory variable P is included in the data set 322.

In this case, the extraction unit 313 requests the first generation unit 311 to add the explanatory variable P. The first generation unit 311 adds the explanatory variable P to the simulation target and performs the simulation of the electrochemical ammonia synthesis again, thereby generating the updated data set 322 including the explanatory variable P and storing the data set in the storage unit 316.

The causal estimation unit 312 generates a causal graph using the updated data set 322 and extracts the specific condition 323 based on the generated causal graph.

The extraction unit 313 extracts, from the updated data set 322, a subset of data that satisfies a condition for an explanatory variable other than the explanatory variable P among the explanatory variables included in the specific condition 323. Then, the extraction unit 313 generates the variable value set 325 from the extracted subset.

For example, when the volcano plot in FIG. 5A is selected as the similar variable relationship information 324, the explanatory variable P is $\Delta E_{NNH}$. Since $\Delta E_{NNH}$ is not included in the data set 322 in FIG. 8, the first generation unit 311 adds $\Delta E_{NNH}$ to the simulation target, and performs the simulation of the electrochemical ammonia synthesis again, thereby generating updated data set 322.

By updating the data set 322 when the value of the explanatory variable P is not included in the data set 322, it can be determined whether or not the volcano plot 326 including the explanatory variable P can be generated. As a result, it is not necessary to perform determination on the explanatory variables other than the explanatory variable P included in the specific condition 323, and trial and error in generating the volcano plot 326 is reduced.

As another possibility, even if the variable value set 325 is divided using the threshold Ta of the explanatory variable P included in the specific condition 323, the volcano plot 326 may not be generated. For example, when the difference between the threshold Ta and the threshold of the explanatory variable P included in the similar variable relationship information 324 is large, the positive and negative signs of the regression coefficient of the group G1 and the positive and negative signs of the regression coefficient of the group G2 become the same, and the volcano plot 326 may not be generated.

Therefore, when the difference from the threshold of the explanatory variable P included in the similar variable relationship information 324 is larger than the predetermined value ε, the extraction unit 313 requests the causal estimation unit 312 to change the threshold for the explanatory variable P. As the predetermined value ε, a value sufficiently smaller than the absolute value of the threshold included in the similar variable relationship information 324 is used.

The causal estimation unit 312 compares the threshold used for multi-level conversion of the explanatory variable P with the threshold included in the similar variable relationship information 324, and changes the threshold used for multi-level conversion so that the difference becomes smaller than a predetermined value ε when the difference between the thresholds is larger than the predetermined value ε. The threshold of the explanatory variable P included in the similar variable relationship information 324 is an example of a first threshold for a predetermined explanatory variable, and the threshold used for multi-level conversion of the explanatory variable P is an example of a second threshold for the predetermined explanatory variable.

Next, the causal estimation unit 312 multi-levels the explanatory variable P using the changed threshold, and generates a condition representing each of the plurality of combinations of explanatory variables again. Then, the causal estimation unit 312 generates a causal graph from the generated conditions, and extracts the specific condition 323 based on the generated causal graph.

The extraction unit 313 generates the variable value set 325 using the extracted specific condition 323, and the second generation unit 314 divides the variable value set 325 into the group G1 and the group G2 using the threshold of the explanatory variable P included in the extracted specific condition 323.

For example, when the volcano plot in FIG. 5A is selected as the similar variable relationship information 324, the explanatory variable P is $\Delta E_{NNH}$, and the threshold of $\Delta E_{NNH}$ is -0.72. In a case where ε = 0.1, the causal estimation unit 312 changes the current threshold such that one of the thresholds used for multi-level conversion of $\Delta E_{NNH}$ is included in the range of $-0.72 \pm 0.1$. For example, the causal estimation unit 312 repeats an operation of halving the interval of the current threshold so that the threshold is included in the range of $-0.72 \pm 0.1$.

For example, in a case where the interval between the current thresholds is 1, and five thresholds of -3, -2, -1, 0, and 1 are used for multi-level conversion of $\Delta E_{NNH}$, none of the thresholds is included in the range of $-0.72 \pm 0.1$. Therefore, when the interval between the thresholds is changed to 0.5 that is a half, nine thresholds of -3, -2.5, 2, -1.5, -1, -0.5, 0, 0.5, and 1 are generated.

However, since none of the nine thresholds is included in the range of $0.72 \pm 0.1$, the interval is changed to 0.25 by further halving the interval between the thresholds. As a result, 17 thresholds of -3, -2.75, -2.5, -2.25, -2, -1.75, -1.5, -1.25, -1, -0.75, -0.5, -0.25, 0, 0.25, 0.5, 0.75, and 1 are generated. In this case, since 0.75 is included in the range of $0.72 \pm 0.1$, the change of the threshold is ended.

By changing the threshold used for multi-level conversion when the difference between the threshold used for multi-level conversion of the explanatory variable P and the threshold of the similar variable relationship information 324 is large, the threshold Ta included in the specific condition 323 is likely to approach the threshold of the similar variable relationship information 324.

As a result, since the volcano plot 326 is easily generated by dividing the variable value set 325 using the threshold Ta, trial and error in generating the volcano plot 326 is further reduced.

FIGS. 11A and 11B are flowcharts illustrating an example of second analysis processing performed by the analysis device 301 of FIG. 3. First, the first generation unit 311 generates the data set 322 by, for example, performing a simulation of electrochemical ammonia synthesis (step 1101).

Next, the causal estimation unit 312 generates a condition representing each of a plurality of combinations of explanatory variables by comprehensively combining the explanatory variables included in the data set 322 (step 1102).

Next, the causal estimation unit 312 extracts a condition having a correlation with the objective variable from the generated conditions (step 1103). Then, the causal estimation unit 312 generates a causal graph for each condition having a correlation with the objective variable, and extracts one or a plurality of specific conditions 323 (step 1104).

Next, the extraction unit 313 selects the similar variable relationship information 324 from the variable relationship information included in the accumulated data 321 (step 1105). Then, the extraction unit 313 checks whether or not the explanatory variable P included in the similar variable relationship information 324 is included in the data set 322 (step 1106).

When the explanatory variable P is included in the data set 322 (step 1106, YES), the extraction unit 313 performs the processing in step 1107 on the specific condition 323 including the explanatory variable P. In step 1107, the extraction unit 313 compares the difference between the threshold Ta of the explanatory variable P included in the specific condition 323 and the threshold of the explanatory variable P included in the similar variable relationship information 324 with the predetermined value ε.

When the difference between the threshold Ta and the threshold of the explanatory variable P included in the similar variable relationship information 324 is equal to or less than the predetermined value ε (step 1107, YES), the extraction unit 313 performs the processing of step 1108. In step 1108, the extraction unit 313 extracts, from the data set 322, a subset of data that satisfies a condition for an explanatory variable other than the explanatory variable P included in the similar variable relationship information 324 among the explanatory variables included in the specific condition 323.

Next, the extraction unit 313 extracts a combination of the value of the explanatory variable P and the value of the objective variable from each data included in the extracted subset, and generates the variable value set 325 including the extracted combination (step 1109).

Next, the second generation unit 314 divides the variable value set 325 into the group G1 and the group G2 using the threshold Ta, and plots points representing combinations included in the group G1 and the group G2 on the XY plane (step 1110). Then, the second generation unit 314 obtains a regression line from the plotted points for each of the group G1 and the group G2, and calculates a regression coefficient (step 1111).

Next, the second generation unit 314 compares the positive and negative signs of the regression coefficient of the group G1 with the positive and negative signs of the regression coefficient of the group G2 (step 1112).

When the positive and negative signs of the regression coefficient of the group G1 are different from the positive and negative signs of the regression coefficient of the group G2 (Step 1112, NO), the second generation unit 314 generates the volcano plot 326 using the variable value set 325 (step 1113).

Next, the second generation unit 314 generates the explanatory information 327 regarding the difference between the volcano plot 326 and the similar variable relationship information 324 (step 1114). Then, the output unit 315 outputs the generated volcano plot 326 and the explanatory information 327 (step 1115).

When the positive and negative signs of the regression coefficient of the group G1 are the same as the positive and negative signs of the regression coefficient of the group G2 (step 1112, YES), the analysis device 301 repeats the processing in and after step 1107 for the next specific condition 323 including the explanatory variable P.

When the difference between the threshold Ta and the threshold of the explanatory variable P included in the similar variable relationship information 324 is larger than the predetermined value ε (step 1107, NO), the extraction unit 313 requests the causal estimation unit 312 to change the threshold for the explanatory variable P. The causal estimation unit 312 changes the threshold used for multi-level conversion of the explanatory variable P (step 1117), and the analysis device 301 repeats the processing of step 1102 and subsequent steps.

When the explanatory variable P is not included in the data set 322 (Step 1106, NO), the extraction unit 313 requests the first generation unit 311 to add the explanatory variable P. The first generation unit 311 adds the explanatory variable P to the simulation target (step 1116), and the analysis device 301 repeats the processing of step 1101 and subsequent steps.

The configurations of the analysis device 101 of FIG. 1 and the analysis device 301 of FIG. 3 are merely examples, and some components may be omitted or changed according to the application or condition of the analysis device.

The flowcharts of FIGS. 2, 11A, and 11B are merely examples, and some processing may be omitted or changed according to the configurations or conditions of the analysis device 101 and the analysis device 301. The analysis processing of FIG. 6 is merely an example, and some processing may be omitted or changed according to the configuration or conditions of the analysis device 301.

The volcano plot and the conditional causal graph illustrated in FIGS. 4A to 5B are merely examples, and the volcano plot and the conditional causal graph included in the accumulated data 321 change according to the data set used in the data processing.

The initial atomic structure illustrated in FIG. 7 is merely an example, and the initial atomic structure changes according to the reaction in the material search and the type of the search target. The data set 322 illustrated in FIG. 8 is merely an example, and the data set 322 changes according to the simulation result.

The volcano plot 326 illustrated in FIG. 9 is merely an example, and the volcano plot 326 changes according to the data set 322 and the similar variable relationship information 324. The causal graph illustrated in FIG. 10 is merely an example, and the causal graph used for generating the explanatory information 327 changes according to the data set 322.

FIG. 12 illustrates a hardware configuration example of an information processing device (computer) used as the analysis device 101 of FIG. 1 and the analysis device 301 of FIG. 3. The information processing device in FIG. 12 includes a central processing unit (CPU) 1201, a memory 1202, an input device 1203, an output device 1204, an auxiliary storage device 1205, a medium driving device 1206, and a network connection device 1207.

These components are hardware and are connected to each other by a bus 1208.

The memory 1202 is, for example, a semiconductor memory such as a read only memory (ROM) and a random access memory (RAM), and stores programs and data used for processing.

The memory 1202 may operate as the storage unit 316 in FIG. 3.

The CPU 1201 (processor) operates as the condition extraction unit 111, the data extraction unit 112, and the comparison unit 113 in FIG. 1, for example, by executing a program using the memory 1202. The CPU 1201 also operates as the first generation unit 311, the causal estimation unit 312, the extraction unit 313, and the second generation unit 314 in FIG. 3 by executing a program using the memory 1202.

The input device 1203 is, for example, a keyboard, a pointing device, or the like, and is used for inputting an instruction or information from a user or an operator. The output device 1204 is, for example, a display device, a printer, or the like, and is used for an inquiry or an instruction to a user or an operator, and outputting a processing result. The output device 1204 may operate as the output unit 315 of FIG. 3. The processing result may be the volcano plot 326 and the explanatory information 327.

The auxiliary storage device 1205 is, for example, a magnetic disk device, an optical disk device, a magneto-optical disk device, a tape device, or the like. The auxiliary storage device 1205 may be a hard disk drive or a solid state drive (SSD). The information processing device can store programs and data in the auxiliary storage device 1205 and load the programs and data into the memory 1202 for use.

The auxiliary storage device 1205 may operate as the storage unit 316 in FIG. 3.

The medium driving device 1206 drives the portable recording medium 1209 and accesses the recorded contents. The portable recording medium 1209 is a memory device, a flexible disk, an optical disk, a magneto-optical disk, or the like. The portable recording medium 1209 may be a compact disk read only memory (CD-ROM), a digital versatile disk (DVD), a universal serial bus (USB) memory, or the like.

The user or the operator can store programs and data in the portable recording medium 1209 and load the programs and data into the memory 1202 for use.

As described above, the computer-readable recording medium that stores the program and data used for processing is a physical (non-transitory) recording medium such as the memory 1202, the auxiliary storage device 1205, or the portable recording medium 1209.

The network connection device 1207 is a communication device that is connected to a communication network such as a wide area network (WAN) or a local area network (LAN) and performs data conversion accompanying communication.

The information processing device can receive programs and data from an external device via the network connection device 1207, load the programs and data into the memory 1202, and use the programs and data. The network connection device 1207 may operate as the output unit 315 in FIG. 3.

Note that the information processing device does not need to include all the components in FIG. 12, and some components may be omitted or changed according to the application or condition of the information processing device. For example, in a case where an interface with a user or an operator is unnecessary, the input device 1203 and the output device 1204 can be omitted. When the portable recording medium 1209 or the communication network is not used, the medium driving device 1206 or the network connection device 1207 can be omitted.

Although the disclosed embodiments and their advantages have been described in detail, those skilled in the art will be able to make various changes, additions, and omissions without departing from the scope of the invention as clearly set forth in the claims.

According to one aspect, a relationship between two variables can be efficiently determined from a set of data including values of a plurality of variables.

## Claims

**1.** An analysis program that causes a computer to execute a process comprising:

first extracting (S201), from a set of data including a value of each of a plurality of explanatory variables and a value of an objective variable, a specific condition having a specific correlation with the objective variable among conditions for at least a part of the plurality of explanatory variables;

second extracting (S202) a set of combinations of a value of a predetermined explanatory variable and a value of the objective variable indicated by predetermined variable relationship information from the set of data based on the specific condition;

dividing (S203) the set of combinations into a first group and a second group; and

comparing (S204) positive and negative signs of a first coefficient indicating a relationship between the predetermined explanatory variable and the objective variable represented by a combination of the first group with positive and negative signs of a second coefficient indicating a relationship between the predetermined explanatory variable and the objective variable represented by a combination of the second group;

wherein the predetermined variable relationship information indicates a relationship between the predetermined explanatory variable and the objective variable in data processing similar to data processing on the set of data.

**2.** The analysis program according to claim 1, wherein the process further includes generating (S1113) specific variable relationship information indicating a relationship between the predetermined explanatory variable and the objective variable in the data processing on the set of data by using the set of combinations when the positive and negative signs of the first coefficient are different from the positive and negative signs of the second coefficient.

**3.** The analysis program according to claim 2, wherein the process further includes generating (S1113) explanatory information on a difference between the specific variable relationship information and the predetermined variable relationship information.

**4.** The analysis program according to claim 2, wherein the predetermined variable relationship information is a volcano plot or a conditional causal graph, and the specific variable relationship information is a volcano plot.

**5.** The analysis program according to any one of claims 1 to 4, wherein the specific correlation indicates that an index indicating strength of a causal relationship between any of the plurality of explanatory variables and the objective variable is equal to or greater than a reference value in a causal graph generated from a condition for at least a part of the plurality of explanatory variables.

**6.** The analysis program according to any one of claims 1 to 4, wherein
the second extracting (S202) includes:

extracting, from the set of data, data that satisfies a condition for an explanatory variable other than the predetermined explanatory variable among the explanatory variables included in the specific condition; and
extracting a combination of a value of the predetermined explanatory variable and a value of the objective variable from data that satisfies the condition for the explanatory variable other than the predetermined explanatory variable.

**7.** The analysis program according to any one of claims 1 to 4, wherein

the process further includes selecting (S1105) the predetermined variable relationship information from a plurality of pieces of variable relationship information, wherein
each of the plurality of pieces of variable relationship information is generated from the set of data including the value of each of the plurality of explanatory variables and the value of the objective variable, and represents a relationship between any of the explanatory variables and the objective variable.

**8.** The analysis program according to any one of claims 1 to 4, wherein

the process further includes updating (S1106, S1116) the set of data so that data including the value of each of the plurality of explanatory variables, the value of the objective variable, and the value of the predetermined explanatory variable is included in the set of data when the value of the predetermined explanatory variable is not included in the set of data, wherein
the second extracting (S202) includes extracting the set of combinations from the updated set of data.

**9.** The analysis program according to any one of claims 1 to 4, wherein

the predetermined variable relationship information includes a first threshold for the predetermined explanatory variable, and
the first extracting (S201) includes extracting a condition for at least a part of the plurality of explanatory variables from the set of data based on a second threshold for the predetermined explanatory variable, and
when a difference between the second threshold and the first threshold is larger than a predetermined value, the computer is further caused to execute processing of changing the second threshold so that the difference between the second threshold and the first threshold is smaller than the predetermined value.

**10.** An analysis device (101, 103) comprising:

a condition extraction unit (111) configured to extract, from a set of data including a value of each of a plurality of explanatory variables and a value of an objective variable, a specific condition having a specific correlation with the objective variable among conditions for at least a part of the plurality of explanatory variables;
a data extraction unit (112) configured to extract a set of combinations of a value of a predetermined explanatory variable and a value of the objective variable indicated by predetermined variable relationship information from the set of data based on the specific condition; and
a comparison unit (113) configured to divide the set of combinations into a first group and a second group, and compares positive and negative signs of a first coefficient indicating a relationship between the predetermined explanatory variable and the objective variable represented by a combination of the first group with positive and negative signs of a second coefficient indicating a relationship between the predetermined explanatory variable and the objective variable represented by a combination of the second group; wherein

the predetermined variable relationship information indicates a relationship between the predetermined explanatory variable and the objective variable in data processing similar to data processing on the set of data.

**11.** The analysis device (101, 103) according to claim 10, wherein the comparison unit (113) is further configured to generate specific variable relationship information indicating a relationship between the predetermined explanatory variable and the objective variable in data processing on the set of data by using the set of combinations when the positive and negative signs of the first coefficient are different from the positive and negative signs of the second coefficient.

**12.** An analysis method carried out by a computer, comprising:

first extracting (S201), from a set of data including a value of each of a plurality of explanatory variables and a value of an objective variable, a specific condition having a specific correlation with the objective variable among conditions for at least a part of the plurality of explanatory variables;
second extracting (S202) a set of combinations of a value of a predetermined explanatory variable and a value of the objective variable indicated by predetermined variable relationship information from the set of data based on the specific condition;
dividing (S203) the set of combinations into a first group and a second group; and
comparing (S204) positive and negative signs of a first coefficient indicating a relationship between the predetermined explanatory variable and the objective variable represented by a combination of the first group with positive and negative signs of a second coefficient indicating a relationship between the predetermined explanatory variable and the objective variable represented by a combination of the second group;
wherein the predetermined variable relationship information indicates a relationship between the predetermined explanatory variable and the objective variable in data processing similar to data processing on the set of data.

**13.** The analysis method according to claim 12, wherein the analysis method includes generating (S1113) specific variable relationship information indicating a relationship between the predetermined explanatory variable and the objective variable in data processing on the set of data by using the set of combinations when the positive and negative signs of the first coefficient are different from the positive and negative signs of the second coefficient.

FIG.1

101

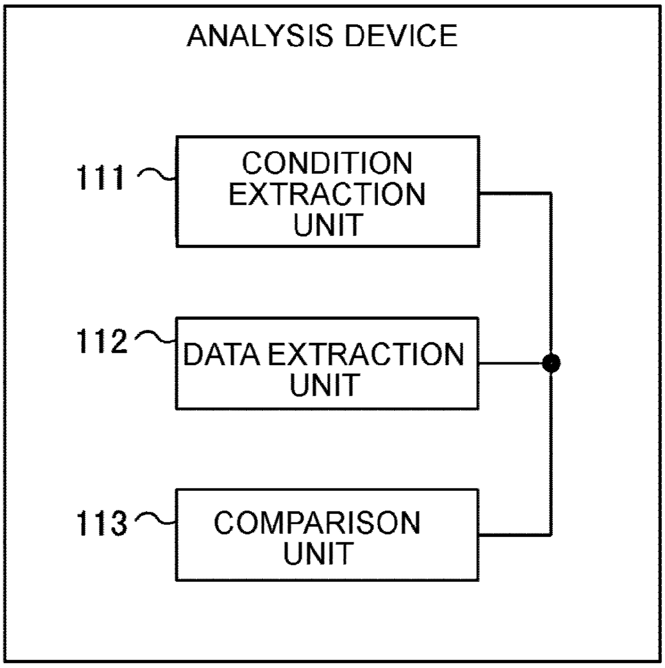


FIG.2

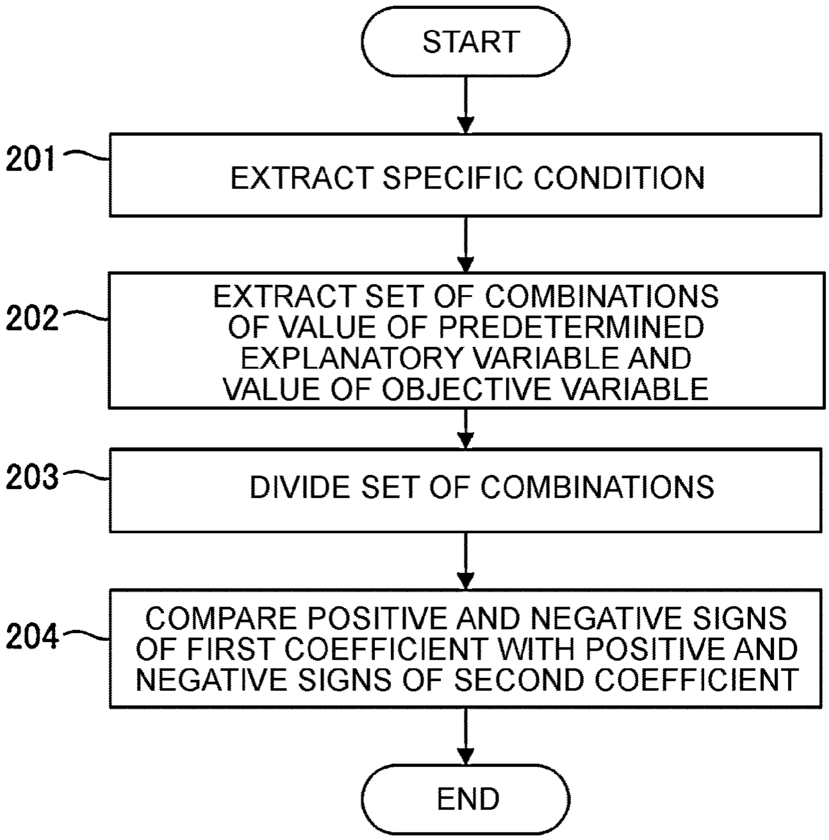

FIG.3

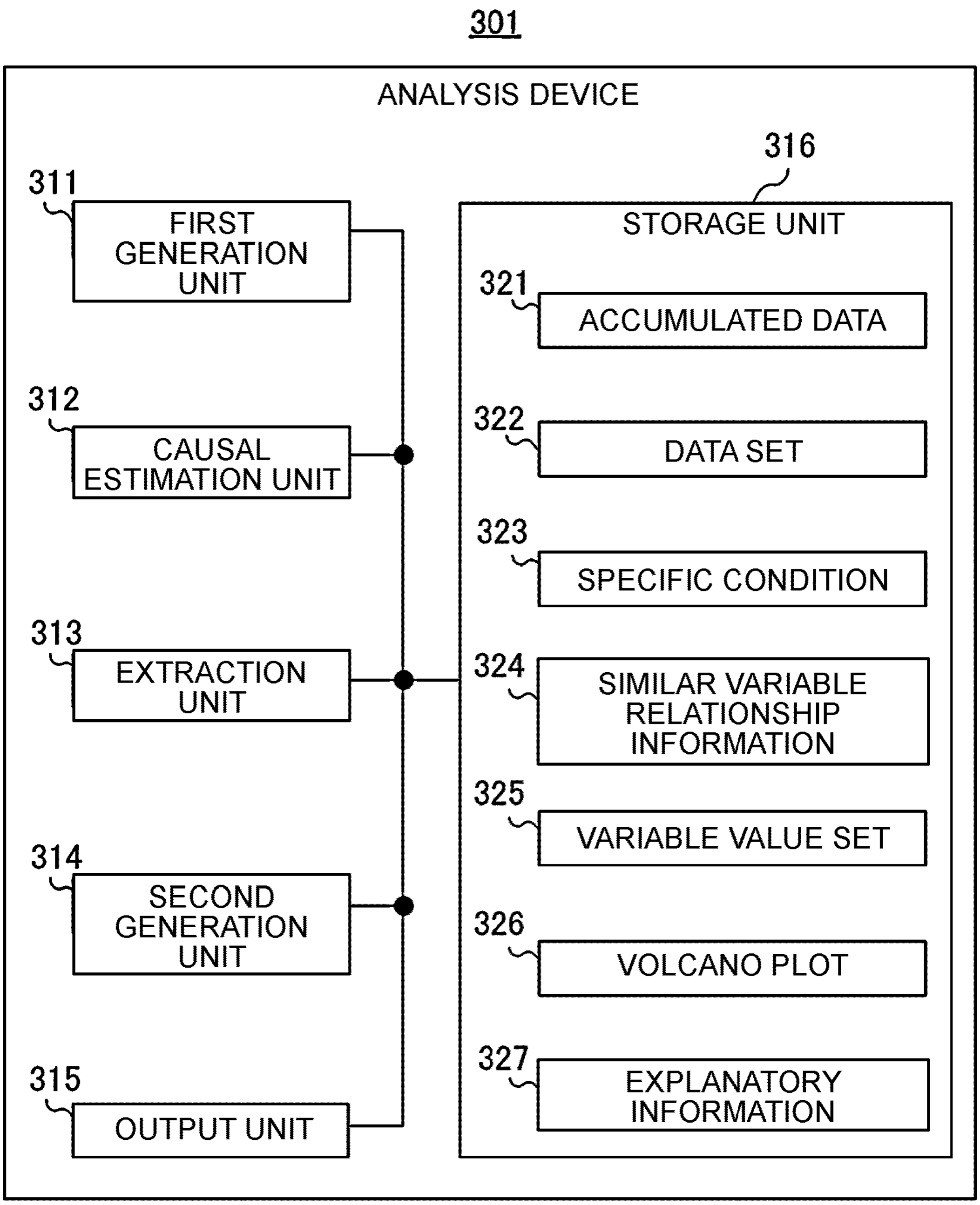
301
ANALYSIS DEVICE
311
FIRST GENERATION UNIT
312
CAUSAL ESTIMATION UNIT
313
EXTRACTION UNIT
314
SECOND GENERATION UNIT
315
OUTPUT UNIT
316
STORAGE UNIT
321
ACCUMULATED DATA
322
DATA SET
323
SPECIFIC CONDITION
324
SIMILAR VARIABLE RELATIONSHIP INFORMATION
325
VARIABLE VALUE SET
326
VOLCANO PLOT
327
EXPLANATORY INFORMATION

FIG.4

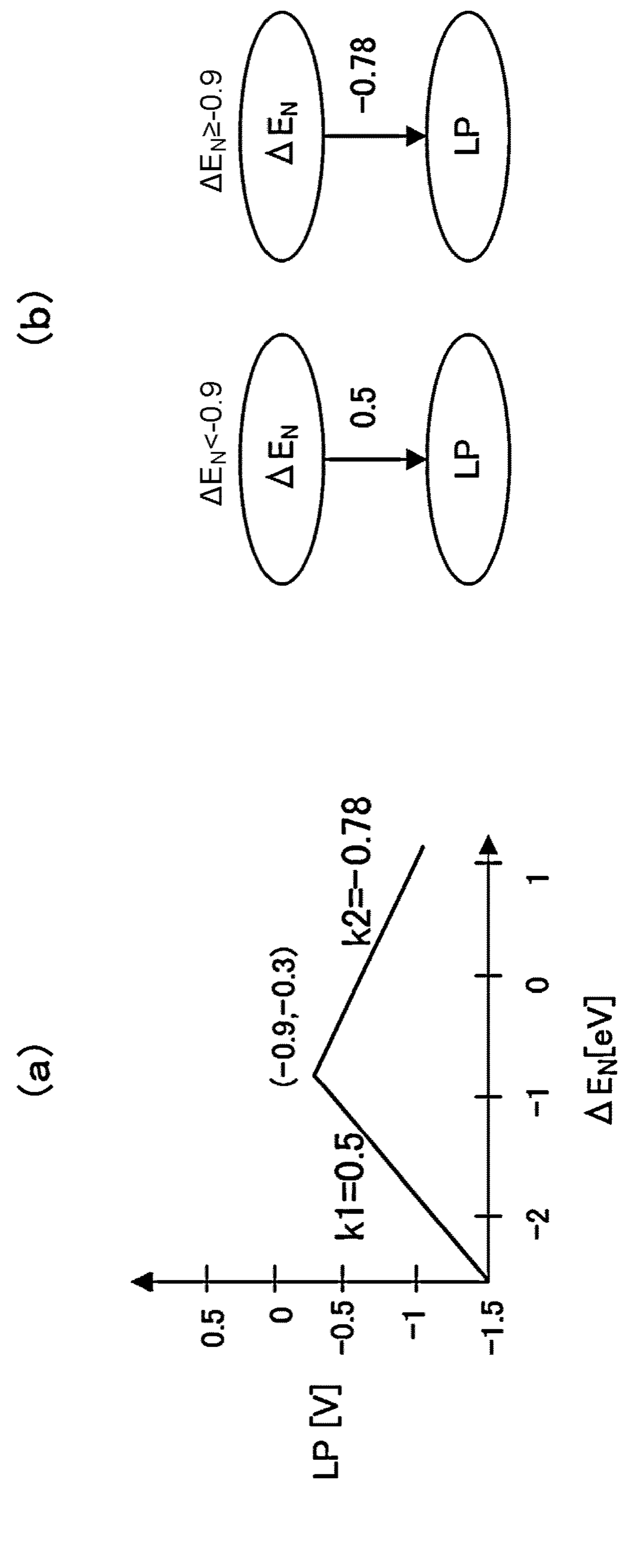
(a)
LP [V]
0.5
0
-0.5
-1
-1.5
-2
-1
0
1
$\Delta E_N$[eV]
k1=0.5
k2=-0.78
(-0.9,-0.3)
(b)
$\Delta E_N$<-0.9
$\Delta E_N$
0.5
LP
$\Delta E_N$≥-0.9
$\Delta E_N$
-0.78
LP

FIG.5

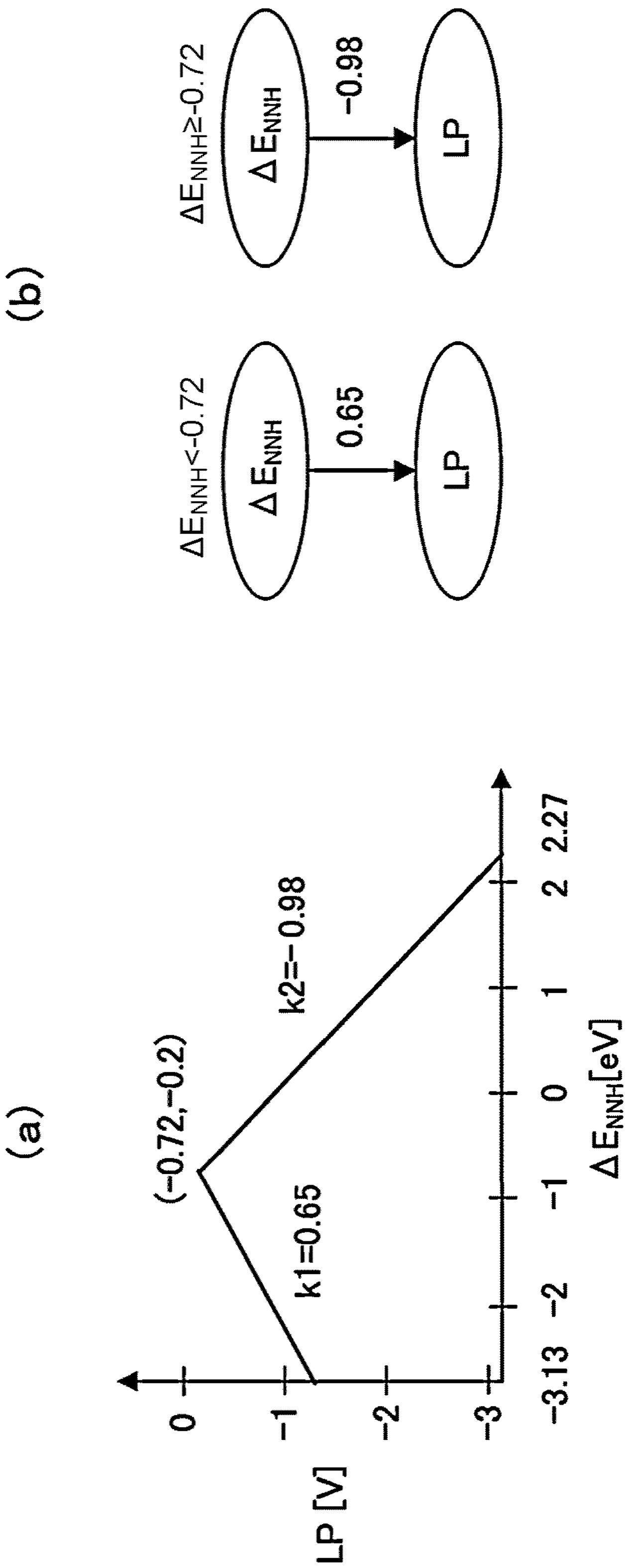
(a)
LP [V]
0
−1
−2
−3
−3.13
−2
−1
0
1
2
2.27
Δ E$_{NNH}$[eV]
k1=0.65
k2=−0.98
(−0.72,−0.2)
(b)
ΔE$_{NNH}$<-0.72
Δ E$_{NNH}$
0.65
LP
ΔE$_{NNH}$≥-0.72
Δ E$_{NNH}$
−0.98
LP

FIG.6

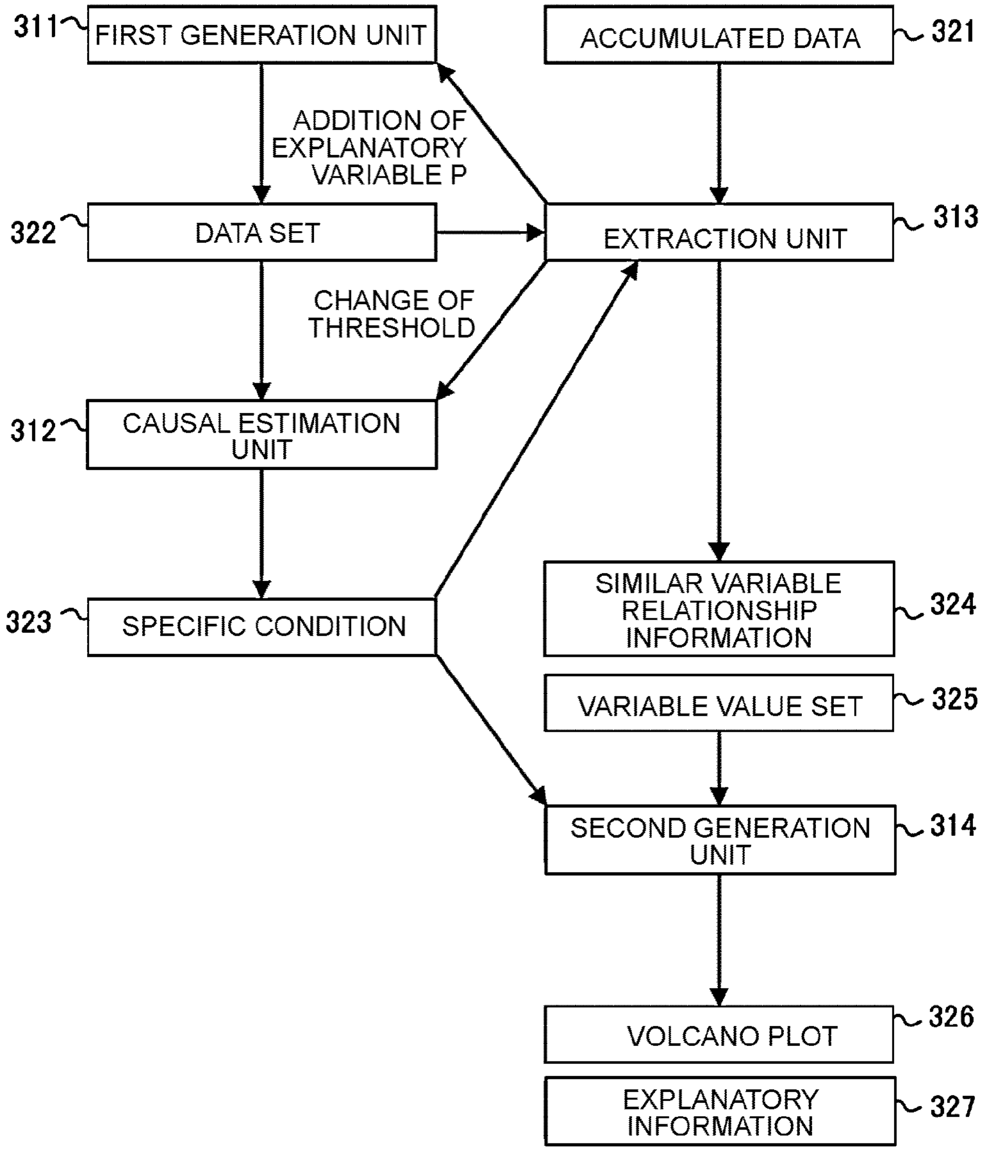
311
FIRST GENERATION UNIT
ACCUMULATED DATA
321
ADDITION OF EXPLANATORY VARIABLE P
322
DATA SET
EXTRACTION UNIT
313
CHANGE OF THRESHOLD
312
CAUSAL ESTIMATION UNIT
323
SPECIFIC CONDITION
SIMILAR VARIABLE RELATIONSHIP INFORMATION
324
VARIABLE VALUE SET
325
SECOND GENERATION UNIT
314
VOLCANO PLOT
326
EXPLANATORY INFORMATION
327

FIG.7

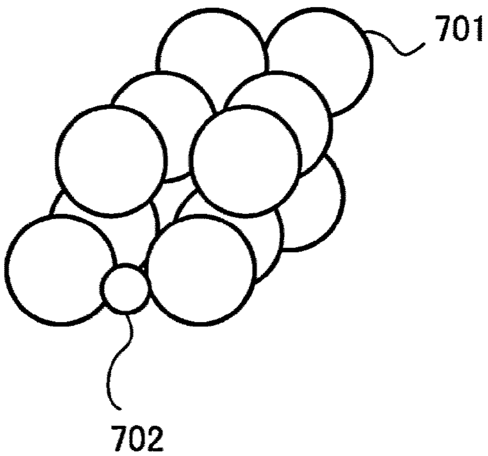
701
702

FIG.8

| ELE-MENT | ATOMIC NUMBER | PERIOD NUMBER | GROUP NUMBER | $\Delta E_N$[eV] | LP[V] |
|---|---|---|---|---|---|
| Al | 13 | 3 | 13 | −2.7 | −1.0 |
| Ba | 56 | 6 | 2 | −1.7 | −0.7 |
| B | 5 | 2 | 13 | −2.4 | −0.9 |
| Be | 4 | 2 | 2 | −2.5 | −1.0 |
| Ca | 20 | 4 | 2 | −2.0 | −0.8 |
| Ce | 58 | 6 | 3 | −2.8 | −1.0 |
| Co | 27 | 4 | 9 | −0.3 | −1.4 |
| ⋮ | ⋮ | ⋮ | ⋮ | ⋮ | ⋮ |

FIG.9

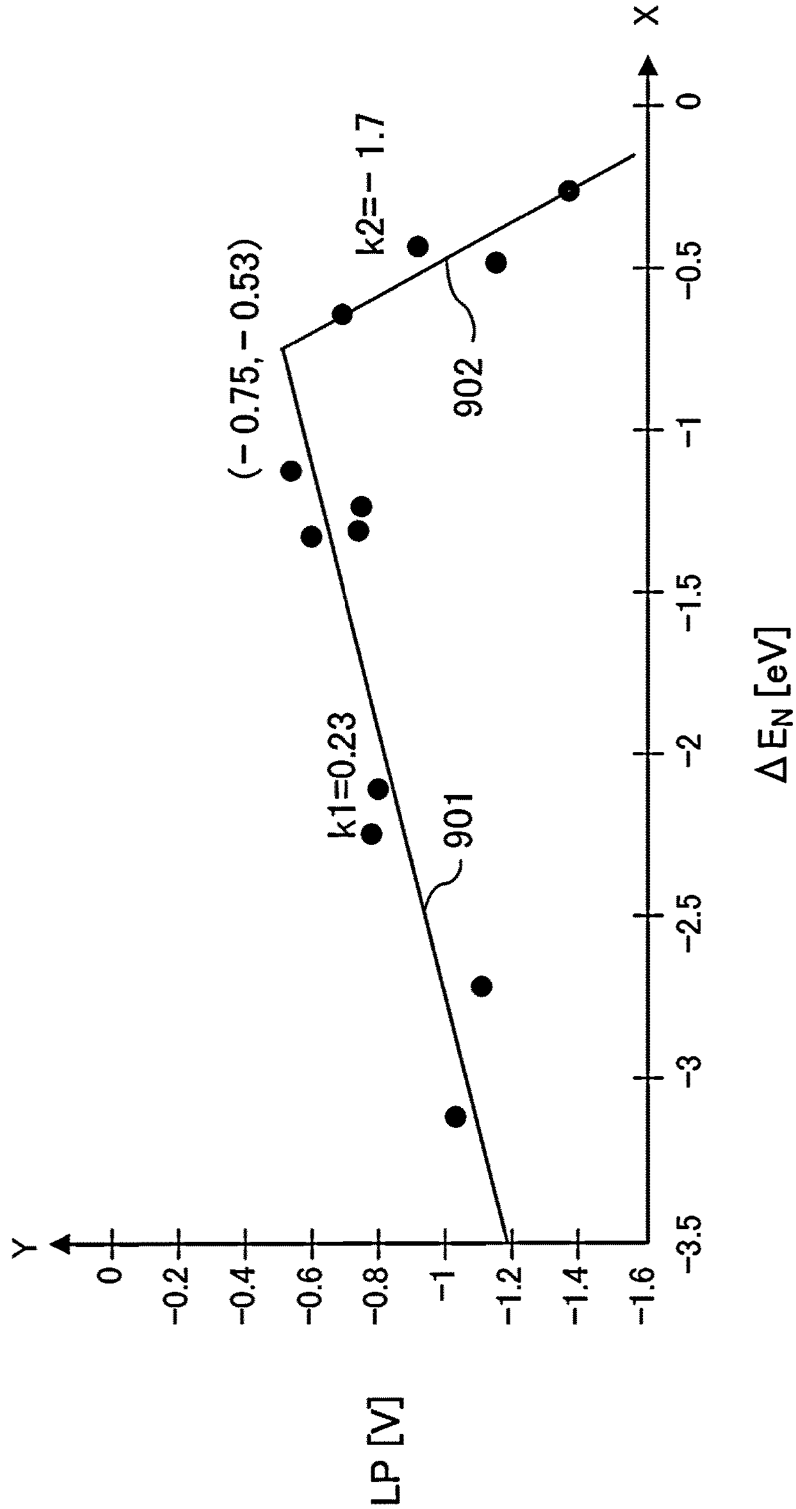
LP [V]
Y
0
-0.2
-0.4
-0.6
-0.8
-1
-1.2
-1.4
-1.6
X
ΔE$_N$ [eV]
-3.5
-3
-2.5
-2
-1.5
-1
-0.5
0
k1=0.23
901
(−0.75, −0.53)
k2=− 1.7
902

FIG.10

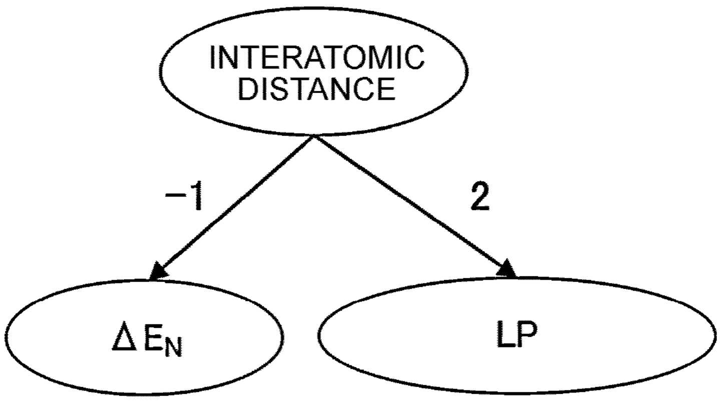

FIG.11A

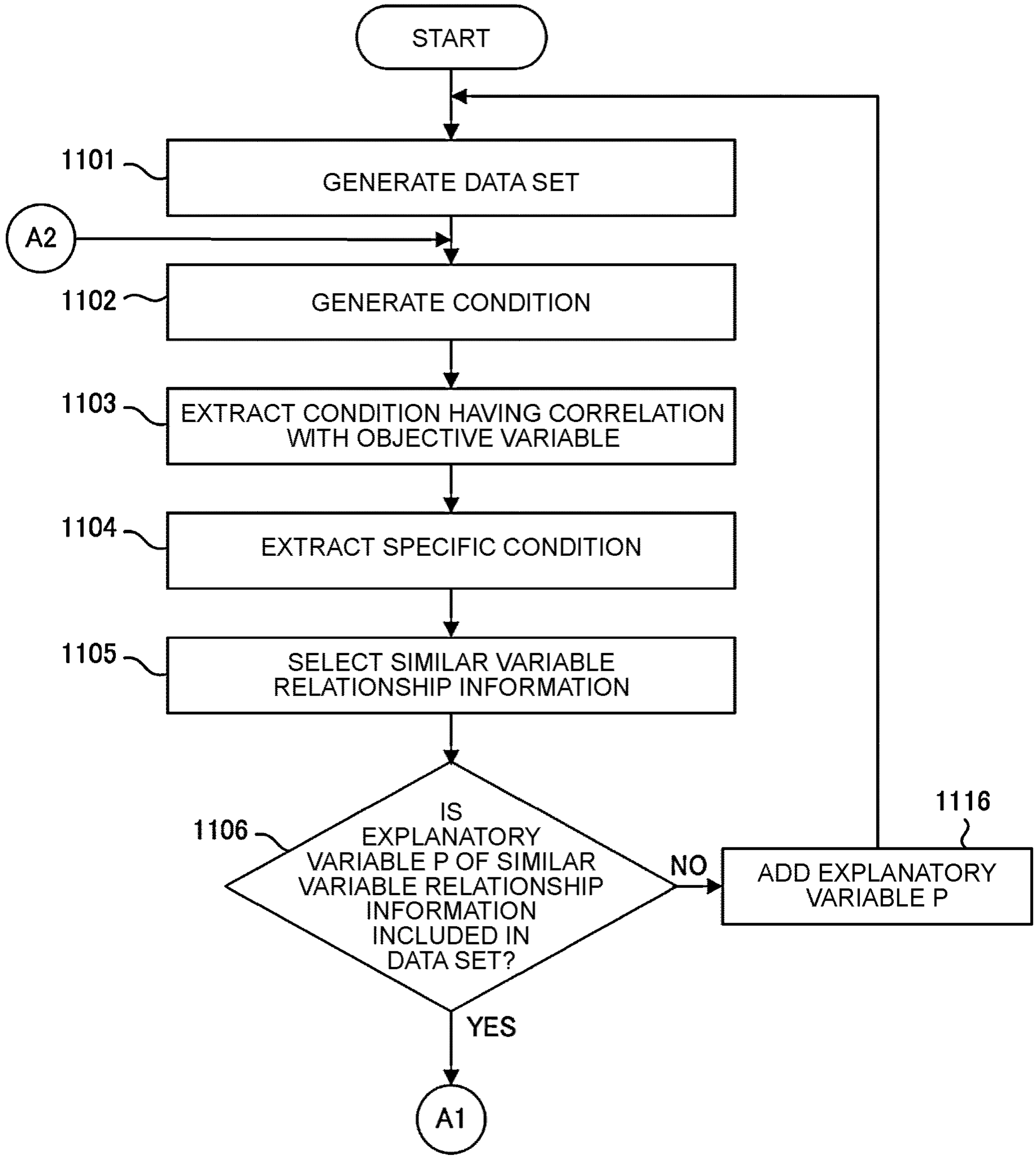
START
1101
GENERATE DATA SET
A2
1102
GENERATE CONDITION
1103
EXTRACT CONDITION HAVING CORRELATION WITH OBJECTIVE VARIABLE
1104
EXTRACT SPECIFIC CONDITION
1105
SELECT SIMILAR VARIABLE RELATIONSHIP INFORMATION
1106
IS EXPLANATORY VARIABLE P OF SIMILAR VARIABLE RELATIONSHIP INFORMATION INCLUDED IN DATA SET?
NO
1116
ADD EXPLANATORY VARIABLE P
YES
A1

FIG.11B

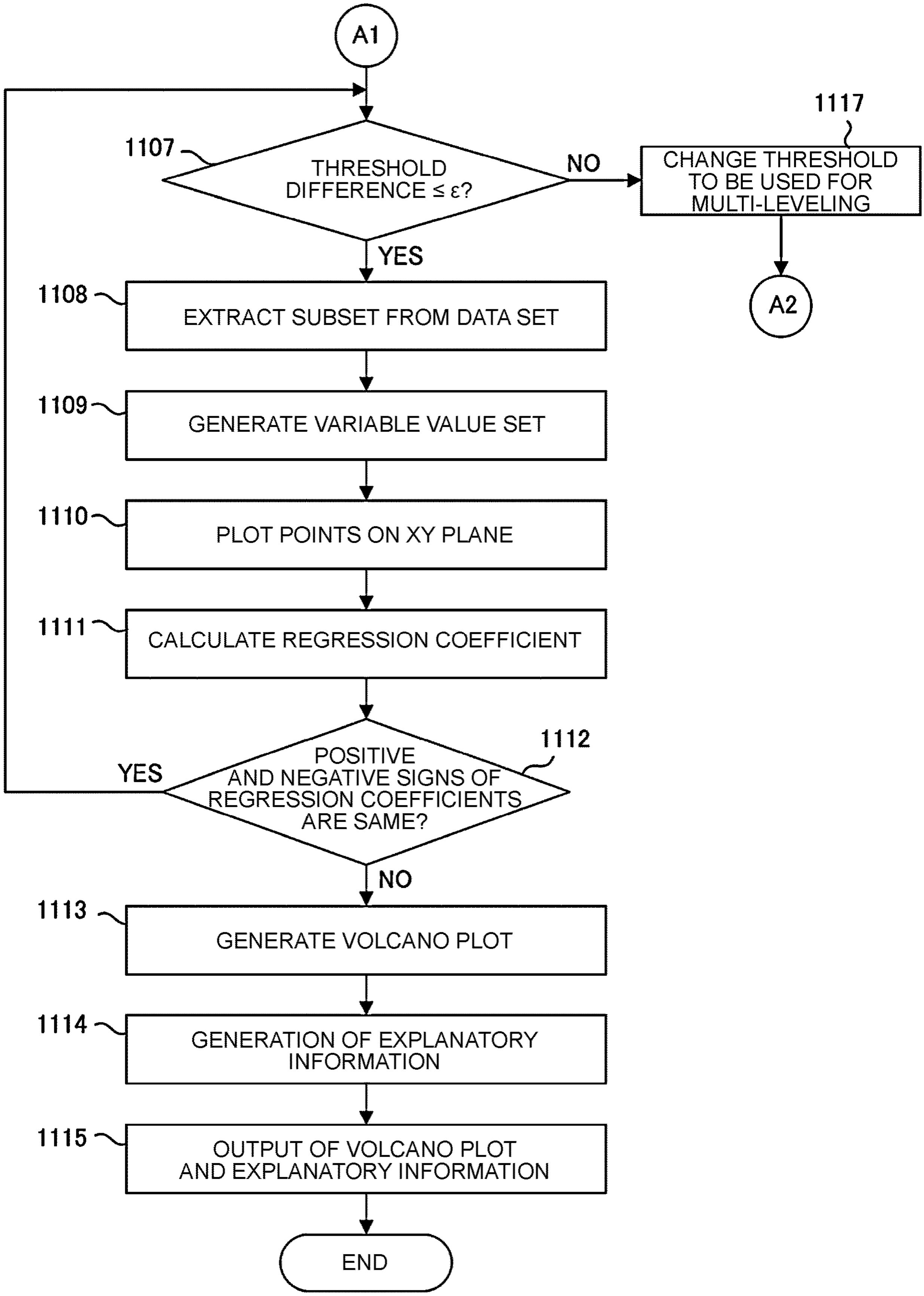
A1
1107
THRESHOLD DIFFERENCE ≤ ε?
NO
1117
CHANGE THRESHOLD TO BE USED FOR MULTI-LEVELING
A2
YES
1108
EXTRACT SUBSET FROM DATA SET
1109
GENERATE VARIABLE VALUE SET
1110
PLOT POINTS ON XY PLANE
1111
CALCULATE REGRESSION COEFFICIENT
1112
POSITIVE AND NEGATIVE SIGNS OF REGRESSION COEFFICIENTS ARE SAME?
YES
NO
1113
GENERATE VOLCANO PLOT
1114
GENERATION OF EXPLANATORY INFORMATION
1115
OUTPUT OF VOLCANO PLOT AND EXPLANATORY INFORMATION
END

FIG.12

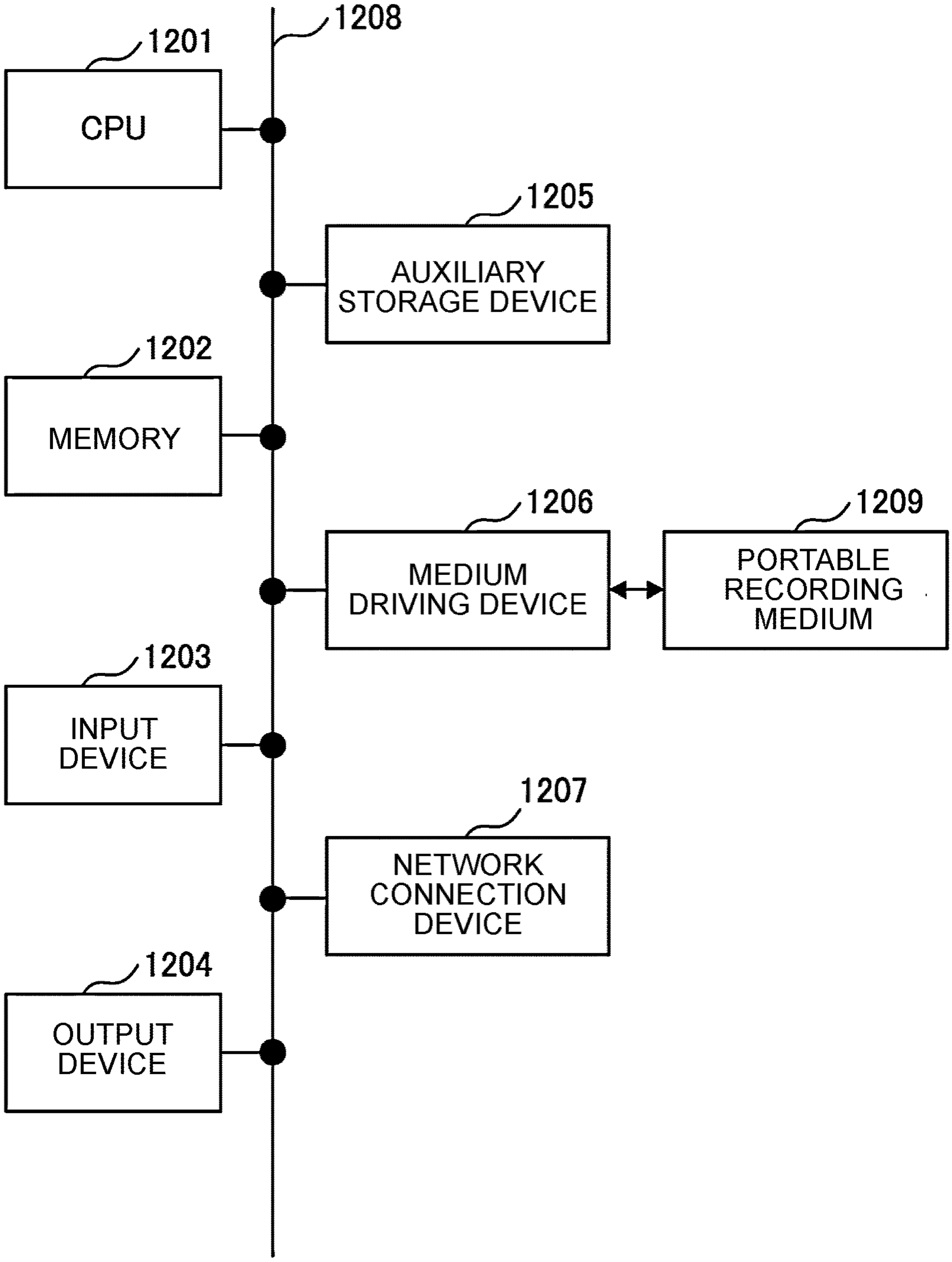
1208
1201
CPU
1205
AUXILIARY STORAGE DEVICE
1202
MEMORY
1206
MEDIUM DRIVING DEVICE
1209
PORTABLE RECORDING MEDIUM
1203
INPUT DEVICE
1207
NETWORK CONNECTION DEVICE
1204
OUTPUT DEVICE

Europäisches Patentamt
European Patent Office
Office européen des brevets

# EUROPEAN SEARCH REPORT

Application Number
EP 25 21 0938

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/018545 A1 (UNIV NEW YORK STATE RES FOUND [US]) 24 January 2019 (2019-01-24)<br>* abstract *<br>* paragraph [0010] - paragraph [0016] *<br>* paragraph [0031] - paragraph [0195] *<br>* figures 1-5 *<br>----- | 1-13 | INV.<br>G06Q10/04<br>G16C20/10 |
| A | US 2021/124740 A1 (ISOZAKI TAKASHI [JP]) 29 April 2021 (2021-04-29)<br>* abstract *<br>* paragraph [0007] - paragraph [0206] *<br>* figures 1-10 *<br>----- | 1-13 | |
| A | WENTIAN LI: "Application of Volcano Plots in Analyses of mRNA Differential Expressions with Microarrays",<br>ARXIV.ORG, CORNELL UNIVERSITY LIBRARY, 201 OLIN LIBRARY CORNELL UNIVERSITY ITHACA, NY 14853,<br>17 March 2011 (2011-03-17), XP080544649,<br>DOI: 10.1142/S0219720012310038<br>* the whole document *<br>----- | 1-13 | |
| | | | TECHNICAL FIELDS SEARCHED (IPC) |
| | | | G06Q<br>G16C |

The present search report has been drawn up for all claims

| Place of search | Date of completion of the search | Examiner |
|---|---|---|
| Munich | 20 March 2026 | Körbler, Günther |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 25 21 0938

This annex lists the patent family members relating to the patent documents cited in the above-mentioned European search report.
The members are as contained in the European Patent Office EDP file on
The European Patent Office is in no way liable for these particulars which are merely given for the purpose of information.

20-03-2026

| Patent document cited in search report | Publication date | Patent family member(s) | Publication date |
|---|---|---|---|
| WO 2019018545 A1 | 24-01-2019 | EP 3655545 A1 | 27-05-2020 |
| | | JP 7195033 B2 | 23-12-2022 |
| | | JP 2020527353 A | 10-09-2020 |
| | | US 2020199676 A1 | 25-06-2020 |
| | | WO 2019018545 A1 | 24-01-2019 |
| US 2021124740 A1 | 29-04-2021 | CN 112368720 A | 12-02-2021 |
| | | EP 3816875 A1 | 05-05-2021 |
| | | JP 7359144 B2 | 11-10-2023 |
| | | JP WO2020004154 A1 | 08-07-2021 |
| | | US 2021124740 A1 | 29-04-2021 |
| | | WO 2020004154 A1 | 02-01-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **E. DRAZEVIC et al.** Are There Any Overlooked Catalysts for Electrochemical NH3 Synthesis-New Insights from Analysis of Thermochemical Data. *iScience*, 18 December 2020, vol. 23 (33)